# EUROPEAN PATENT SPECIFICATION

(11) **EP 3 303 624 B1**
(45) Date of publication and mention of the grant of the patent: **19.05.2021**
(21) Application number: 16729798.5
(22) Date of filing: 25.05.2016
(51) Int. Cl.: C12Q 1/6827

(54) **METHODS FOR ENHANCED CGH ANALYSIS**
VERFAHREN ZUR VERBESSERTEN CGH-ANALYSE
PROCÉDÉS POUR L'ANALYSE AMÉLIORÉE D'UN SIMULATEUR CGH

(30) Priority: 29.05.2015 EP 15169947
(43) Date of publication of application: 11.04.2018
(73) Proprietor: Altergon S.A., 6901 Lugano (CH)
(72) Inventor: IANNONE, Mariano, 6901 Lugano (CH); AMOROSI, Stefania, 6901 Lugano (CH); PICCININNI, Sabina, 6901 Lugano (CH); FOSSATI, Tiziano, 6901 Lugano (CH)
(74) Representative: Gerli, Paolo
(86) International application number: PCT/EP2016/061764
(87) International publication number: WO 2016/193084

(56) References cited:
- WO-A2-2009/062166
- US-A1- 2008 026 390
- SVETLANA A. YATSENKO ET AL: "Microarray-Based Comparative Genomic Hybridization Using Sex-Matched Reference DNA Provides Greater Sensitivity for Detection of Sex Chromosome Imbalances than Array-Comparative Genomic Hybridization with Sex-Mismatched Reference DNA", JOURNAL OF MOLECULAR DIAGNOSTICS,THE, vol. 11, no. 3, 1 May 2009 (2009-05-01), pages 226-237, XP055233498, US ISSN: 1525-1578, DOI: 10.2353/jmoldx.2009.080064 cited in the application
- W. R. LAI ET AL: "Comparative analysis of algorithms for identifying amplifications and deletions in array CGH data", BIOINFORMATICS., vol. 21, no. 19, 1 October 2005 (2005-10-01), pages 3763-3770, XP055233480, GB ISSN: 1367-4803, DOI: 10.1093/bioinformatics/bti611
- A. E. DELLINGER ET AL: "Comparative analyses of seven algorithms for copy number variant identification from single nucleotide polymorphism arrays", NUCLEIC ACIDS RESEARCH, vol. 38, no. 9, 1 May 2010 (2010-05-01), pages e105-e105, XP055233486, GB ISSN: 0305-1048, DOI: 10.1093/nar/gkq040 cited in the application
- PINTO D ET AL: "Comprehensive assessment of array-based platforms and calling algorithms for detection of copy number variants", NATURE BIOTECHNOLOGY, NATURE PUBLISHING GROUP, UNITED STATES, vol. 29, no. 6, 1 June 2011 (2011-06-01), pages 512-520, XP002735142, ISSN: 1546-1696, DOI: 10.1038/NBT.1852 [retrieved on 2011-05-08] cited in the application
- H. TÖNNIES ET AL: "First Systematic CGH-based Analyses of Ancient DNA Samples of Malformed Fetuses Preserved in the Meckel Anatomical Collection in Halle/Saale (Germany)", JOURNAL OF HISTOCHEMISTRY AND CYTOCHEMISTRY, vol. 53, no. 3, 26 March 2005 (2005-03-26) , pages 381-384, XP055633200, US ISSN: 0022-1554, DOI: 10.1369/jhc.4B6427.2005

## Description

### FIELD OF THE INVENTION

The present invention belongs to the field of the analysis of chromosomal unbalances via competitive genomic hybridization (CGH).

### BACKGROUND OF THE INVENTION

In the field comparative genomic hybridization (CGH), the introduction of microarrays has provided a distinct advantage over conventional cytogenetic analysis because they have the potential to detect the majority of microscopic and sub microscopic chromosomal abnormalities over the whole genome under investigation.¹ Chromosomal analysis can be performed in one experiment on cells derived from many sources, including peripheral blood lymphocytes or fetal blood, oocytes or derived polar body and spermatozoa, blastomeres of embryos and any associated biopsy, chorionic villi, amniotic fluid cells, skin, bone marrow, fetal tissues (e.g. lung, liver), solid tumors, and ascites. Existing methods involved in this technology, first imply the isolation of the genetic material from those cells.^{2,3}

The high-throughput technology of microarrays offer its potential role also in veterinary medicine for the development of diagnostic and prognostic tests and for the identification of novel therapeutic targets.⁴

Numerous studies demonstrated that an inherited chromosome aberration could impact on reproductive efficacy, compromising the success attained over the years in animal breeding. Over the past 40 years, hundreds of scientific publications reporting original chromosomal abnormalities generally associated with clinical disorders (mainly fertility impairment) have been published. Each year about 8,000 and 10,000 chromosomal analyses are carried out worldwide, mainly in cattle^{5, 6}, pigs⁷, and horses. For instance, cytogenetic analyses of horses have benefited the horse industry by identifying chromosomal aberrations causing congenital abnormalities, embryonic loss and infertility, this highlights the recent concepts of chromosomal roles also in animal reproduction.^{8, 9} Moreover, many human and animal diseases share a pathogenetic basis and so they represent good comparative models for human disease arising the concept of "one Medicine"; there are special interdependent area of study, with one of the closest being oncology.^{10, 11}

Numerical chromosome irregularities (aneuploidies) are extremely common in cultured staminal, cancer cells and in particular in human oocytes and embryos and are associated with a variety of negative outcomes for both natural cycles and those using assisted conception techniques. Embryos containing the wrong number of chromosomes (aneuploidy) may fail to implant in the uterus, miscarry, or lead to new-borns with serious medical problems. It therefore seems reasonable think to a reliable technique for distinguish the normal from the aneuploidy embryos as a useful tool for physicians and embryologists assisting them for the right choice. This conception has led to the development of a variety of methods for the detection of chromosomal abnormalities in the different samples from the in vitro fertilization (IVF) field, most over referred as PGS (Preimplantation Genetic Screening).^{12,} 13

PGS is a method that seeks to improve the outcomes of assisted reproductive treatments, such as IVF, by ensuring that embryos chosen for the uterus transfer are chromosomally normal. The combination of advances in genetics and embryology seems poised to usher in a new era in the treatment of infertility. In particular, aneuploidy testing is the name used to describe a process of counting the number of chromosomes present in a cell and CGH is a technique that has been employed to detect their presence and identify the genomic location of amplified or deleted DNA sequences. If a cell does not have exactly 46 chromosomes, is distinct as "aneuploid". ^{14, 15, 16}

In addition to the occurrence of extra chromosomes, there may be chromosomes missing from the embryo (GAIN or LOSS is the name to respectively describe each phenomenon). In most cases, either a loss or gain in chromosomes will result in an embryo that will not implant or grow normally. CGH testing of embryos allows us to determine the number of chromosomes in each embryo before they are transferred into the mother's uterus or frozen for future use.

It is possible to test each individual embryo created through IVF and count for the number of chromosomes present. This is done by taking cells from the embryo on either the day 3 or 5 of embryo development and performing tests on those cells to analyse their genetic status. The embryo can easily compensate for the removal of cells with a few cell divisions. In good hands, the biopsy of an embryo has little effect on its growth, although it may slow its growth down slightly.^{17, 18}

Beside PGS, Preimplantation Genetic Diagnosis (PGD) aims to identify embryos free of inherited genetic conditions, attributed either to mutations affecting the function of genes or abnormalities affecting the copy number of whole chromosomes or chromosomal regions. The selection and preferential transfer of genetically normal embryos provides a high probability that any pregnancy established will be healthy, reducing the possibility that termination may need to be considered and, in certain cases, assisting in the eradication of an inherited disease from a family.¹⁹ Both procedures PGS and PGD, involves the generation of embryos using IVF techniques, followed by biopsy and testing of embryonic material.

Because the transfer of a chromosomally abnormal embryo is unlikely to result in a healthy live birth, it has been suggested that efforts should be made to identify and preferentially transfer euploid embryos. In theory, this practice should improve the implantation and pregnancy rates achieved after IVF.

In the past 10 years, an increasing number of fertility clinics have adopted chromosome screening strategies to assist in the identification and transfer of viable embryos. This approach, has most often been targeted at patients considered to have an elevated risk of producing aneuploid oocytes and/or embryos, specifically couples who have experienced repeated implantation failure (RIF), or recurrent pregnancy loss, or where the female partner is of advanced reproductive age (ARA) and thus in the presence of a male factor.^{20, 21, 22}

Even though PGS is closely related to PGD, its clinical application has proven to be far more controversial. During the past few years, debates have arisen concerning the diagnostic value of PGS using Fluorescent *in situ* Hybridisation, the effect of single or double blastomere biopsy on embryonic viability, and the impact of cleavage-stage mosaicism on accurate diagnosis of euploidy/aneuploidy.^{23, 24}

Fluorescent in situ Hybridisation (FISH) has been the method of choice to examine chromosomes in the biopsied material, and various protocols have been described, scoring 5 to 12 chromosomes per oocyte or embryo, it does not permit access to full karyotyping of all 23 chromosome pairs.^{25, 26}

Single Nucleotide Polymorphism (SNP) array technology is distinct from the CGH array and may also be used to determine cells copy number alteration in DNA samples. The mechanism used is different and does not need competitive comparison, the method relies on quantification of individual alleles and subsequent radiometric calculation. Disadvantages include increased noise level, longer protocol, complexity in data interpretation and limited application to diploid samples.²⁷

The recent introduction of more advanced techniques, such as CGH, Spectral Karyotyping, Primed *in situ* Labeling (PRINS), and Peptide Nucleic Acid (PNA) techniques have made karyotyping of all 23 chromosome pairs possible. These new methodologies proved to be rather intensive with respect to time, technology, and costs.²⁸

Besides, the DNA microarray is giving a high-quality support to this assay and provides an invaluable technique for large scale analysis. In the two-channel DNA microarray assay, the DNA isolated from the test cells and normal reference cells is respectively labelled with two distinct fluorescent dyes before being co-hybridized to immobilized DNA probes on a microarray slide. During hybridization, the two fluorescently labelled DNAs (test and reference) compete for hybridization to the immobilized DNA probes strands and the repetitive sequences are removed or reduced by some means. Hybridization reactions between complementary strands occur only between the labelled antisense strand and immobilized sense strand.

The ratio of the intensities of the two fluorescently labelled DNAs is used to quantify the relative levels of genomic regions in the samples. This method serves well for pair-wise comparison of CNV (copy number variations) levels in the samples.²⁹ With over thousand different DNA probes immobilized on the microarray, the relative CNV level of all the chromosomes in the samples can be obtained in a single assay.^{30, 31, 32}

DNA microarrays have found applications in gene discovery, disease diagnosis, cancer, pharmacogenomics and toxicology research. They are increasingly used for a series of related samples, for which a comparison across all samples is desirable. ^{33, 34}

It is very common in gene expression the comparison of treatment versus control samples, where the most natural reference is the wild type or the biological controls, which are often the most abundant. However if the study aims to compare each sample against others, there is no natural control. A reliable alternative is a pooled reference DNA which reduces the number of large errors. Some investigators take this further and created a 'universal reference' derived from several standard cell lines, which they use most often in their experiments. Using a universal reference enables them to compare results for all their experiments. ³⁵

For comparing a number of samples of equal interest and high quality, each sample is hybridized to each of two different samples in two different dye orientations. This design results in half the variance per estimate, because each sample occurs twice, rather than once; at the cost of only one more chip. The drawback is that if one chip fails, or is of poor quality, then the error variance for all estimates is doubled. On the other hand, the fact that only two conditions can be applied to each array complicates the design, either because usually there are more than two conditions, or because it is not recommendable to directly hybridize two samples in one array, because this creates DNA artificial pairings.

The most common design for two color (competitively hybridised spotted) arrays is the 'reference design'. In the simplest way each experimental sample is hybridized against a common reference sample.

While the use of un-amplified genomic DNA as reference is very common in array-CGH, the corresponding application in PGS/PGD results in high noise level because of the lack of balance amongst the two DNA preparation, amplified sample and un-amplified reference. Accordingly, this imbalance increases if a pooled DNA reference is used in this contest.

The reference material often used in this context is a normal pooled DNA sample diluted to an amount broadly comparable to the amount of DNA as a small number of single cells. The corresponding rate of the diluted reference DNA is then amplified in parallel, next to DNA sample. Despite the use of these molecular biology approaches, matching the properties of the test and reference DNA is not always efficient and often the clearness of the results can ambiguously vary. The most common approach to demonstrate successful functioning of an experiment in diagnostic application is the use of internal controls and in individual assay a reference sample with known copy number variations.³⁶

Most often the reference sample is mismatches against the sexual chromosomes and consequently a measure of the dynamic range could be guaranteed. While this approach is affordable in many cases, unfortunately in PGS is not applicable because of the unknown gender of the sample especially when referred to a blastomere or blastocyst biopsy that could be of either sex.

Additionally, Svetlana A. *et al.* in their work demonstrated that microarray-based CGH using sex-matched reference DNA provides greater sensitivity for detection of sex chromosome imbalances than array CGH with sex mismatched reference DNA. Using sex mismatched reference DNAs in array-CGH analysis may generate false positive, false negative and ambiguous results for sex-chromosome specific probes, thus masking potential pathogenic genomic imbalances. Accordingly, the use of a DNA reference carrying known copy number alterations is not recommended because the same alterations in embryo/oocyte are unstable and consequently highly variable.³⁷

Alternatively, there is the possibility to use non-human control sequences, which represents in theory an ideal approach but practically the choice of the genomic sequences able to mimic the human sequences behaviour as to be accurate and additionally they could suffer from the same given amplification biases.

The remaining solution is to analyze the test sample through two conventional CGH experiments one against a male and the second against the female reference DNA an approach that suffer for the high cost of the application.

Buffart et al. ³⁸ suggested as improvement to this technology a modified array-CGH termed as "across array hybridization" where test and sample DNA are compared from different array. Array-CGH was performed using the reference pool analysed on a different channel array. As the second channel within the sample array is no longer used for hybridizing the normal reference DNA, this channel can be used to hybridize a second sample to the same array. This method while offer advantage in terms of costs and potentially in data quality because it removes dye biases, it is not recommendable to directly hybridize two samples in one array because of the occurrence of DNA artificial pairings as said above.

For CGH assay to be applied in this context, amplification of the whole genome is required to increase the small amount of DNA available in cell (5-10 pg) to a level suitable for this analysis. Commonly used methods for amplification include DOP-PCR (diverse random primers strategy) or more recently plexysomes technology libraries kit such us Genomeplex (Rubicon Genomics) or branched-PCR using Phy polymerase kit (Repli-G, Qiagen).

In order to attain optimal results array CGH requires that both DNAs reference and sample have to be equilibrated in terms of quality and concentration. It is noteworthy that in this context the genetic material is the principal point of any analysis because is representative of each cell analysed. Currently, it is possible to isolate the DNA from a polar body, a blastomere (cleavage stage embryo) or a trophectoderm biopsy (5th 6th day of in vitro embryo developmental stage).

Polar bodies are the oocyte counterpart cells ejected in two different moment of an egg life cycle. The polar bodies have no known function except to assist in cell division. They are simply "by-products" of the egg's division. Once implantation occurs, the polar bodies disintegrate and are not part of the developing fetus.³⁹

Embryo biopsy is a technique that is performed during IVF procedures when an embryo has reached six to eight cell stage (about 72 hours or day 3 of embryo culture). One or two cells, or blastomeres, are separated from the rest of the embryo and removed from the zona pellucida which is the shell surrounding the developing embryo. After removal of the cell(s), the developing embryo is placed back into the culture media and returned to the incubator where it can resume its normal growth and development.⁴⁰

Trophoblast biopsy is the newest technique for obtaining cells from a developing embryo for genetic testing. By the 5th day of development, the embryos is divided into approximately 100 cells the inner cell mass and the trophoblast cells. The advantage of trophoblast biopsy is that several cells can be obtained without critical affecting the development of the embryo. By obtaining several cells, more genetic information is available. This will increase the accuracy of the results.⁴¹

However, PGS application requires unique technical challenges, in some embodiments the assessment of chromosomal cell content can be made withdrawing cells after fertilization or measuring indirectly the genetic content assaying polar bodies and thus the oocyte generating the embryo. In other embodiment this application exist with the solely purpose to investigate the oocyte and no embryo is necessarily generated. This is a common procedure where samples are collected in cryo-banks.

The available CGH array methods require a very large number of hybridization tests when aimed at detecting chromosome imbalances in particular cases where the sample sex is not known beforehand (such being the case of e.g. embryos subjected to PGS): this depends from the fact that the test DNA must be hybridized separately with both male and female reference DNAs to get reliable information on possible chromosome unbalances; this duplication of testing is further multiplied by the number of DNA samples be analysed. The need is therefore strongly present for new CGH array methods capable to reduce the number of hybridization tests needed, ideally working with a single array per each test DNA.

The identification of copy number variation has been extensively used to detect aberration of any size across the whole genome, however different discrepancies call from a replicate samples have been reported to date even when measured on the same platform.⁴² In addition, different algorithms can provide different calls on the same sample. A further need is therefore present for new analysis pipelines for CGH array data offering a set of implemented algorithm which can remove array specific biases and automatically select region of gain and losses along the genome reducing the risk of assay failure in terms of false positive (FP) and/or false negative (FN).^{43, 44}

Further variability in CGH testing is caused by the variation in quality of the reference DNA used for competitive hybridization with the test DNA. A further need is therefore present for new CHG methods involving an internal quality control for the reference DNA in use.

### SUMMARY OF THE INVENTION

The present inventors have now devised a new method for determining chromosomes imbalances in a test DNA sample, especially when of unknown sex (i.e. when the sex of the donor of said test DNA is unknown), such being typically the case of embryos undergoing genetic screening prior to implantation. It is to be stated that the method can be used to determine imbalances in both the chromosome types, sexual chromosomes (gonosomes) and non-sexual chromosomes (autosomes), although it is of particularly advantageous when used to determine DNA imbalances in gonosomes. The main feature of the present method, based on CGH array technique, consists in eliminating the need for double hybridizations of the test DNA with dual-sex reference DNAs (i.e. reference DNA from male donor + reference DNA from female donor), until now required. The present method is thus performed using a single hybridization array (herein called "test" hybridization array), on which the test DNA is competitively hybridized against only one reference DNA (from male or female donor, indifferently); the result obtained from said test hybridization array is then combined with the one obtained from a "reference" hybridization array on which reference DNAs obtained from donors of the two opposite sexes have been competitively hybridized. The combination of results from the "test" and the "reference" hybridization arrays, provides the final number of chromosomal imbalances (copy number variations, herein "CNV") within the test DNA It is to be pointed out that the reference hybridization is not repeated *n* times when *n* test DNA samples undergo the method: instead, it can be performed only-once in order to generate the corresponding result: such result, suitably stored, can be further combined with each of the results generated by *n* test DNA samples. Therefore, for each single test DNA to be analyzed, only a single hybridization array is used.

Object of the invention is thus a single array CGH method to assess the presence and number of copy number variations [herein "CNV"] throughout gonosomal and autosomal genomic DNA from a test sample [herein "test-DNA"], via CGH technique, by comparing said test-DNA with a reference genomic DNA from a male (or female) donor [herein "reference-DNA"], said method comprising the following steps:
a) Labeling with a first marker, said test-DNA, thus obtaining a labeled test-DNA;
b) Labeling with a second marker, different from the first, a male (or female) reference-DNA, thus obtaining a labeled male (or female) reference-DNA;
c) Hybridizing the DNAs obtained in steps a) and b) to a test hybridization array, thus obtaining a combined pattern of signal intensities from both markers, deriving from the corresponding hybridized DNA molecules;
d) From the combined pattern of step c), determine the single signal intensities produced by the said first and second marker;
e) Perform a first evaluation of CNV in said test-DNA, by comparing against each other the two signal intensities obtained in step d);
f) Labeling with said first marker, a reference-DNA (sexually opposite to the one used in step b), thus obtaining a reference-DNA labeled with said first marker;
g) Labeling with said second marker, said reference-DNA sexually opposite to the one used in step b), thus obtaining a reference-DNA labeled with said second marker;
h) Hybridizing both DNAs obtained in steps f) and g) to a reference hybridization array, thus obtaining a combined pattern of signal intensities from both markers, deriving from the corresponding hybridized DNA molecules;
i) From the combined pattern of step h), determine the single signal intensities produced by the said first and second marker;
j) Perform a second evaluation of CNV in said test-DNA, by comparing against each other:
   - the signal intensity of said test-DNA labeled with said first marker, as determined in step d),
   - with the signal intensity of said reference-DNA labeled with said second marker as determined in step i);
k) combine the CNV values obtained in steps e) and j), to obtain a final CNV determination, relevant to the gonosomal and autosomal genomic DNA, thus obtaining a final CNV value relevant to the whole genomic DNA subjected to analysis.

The present method allows to cut by half the number of arrays necessary in a support for CGH testing of *n* DNA samples, and consequently, the time needed for such analysis. The disclosure thus extends to new, more compact supports for CGH analysis, wherein *n* test hybridization arrays are provided for testing *n* DNA samples according to steps a)-e) of the present method, and a single reference hybridization array for performing the step h), thus avoiding the duplication of arrays required by the prior art. The disclosure also extends to a kit for performing such method. The disclosure further includes the possibility of reducing the bias in the signal intensities generated by hybridization of the marked DNA molecules, via a suitable data processing. The disclosure further includes an internal quality control of the reference DNA used for hybridization.

### DESCRIPTION OF THE FIGURES

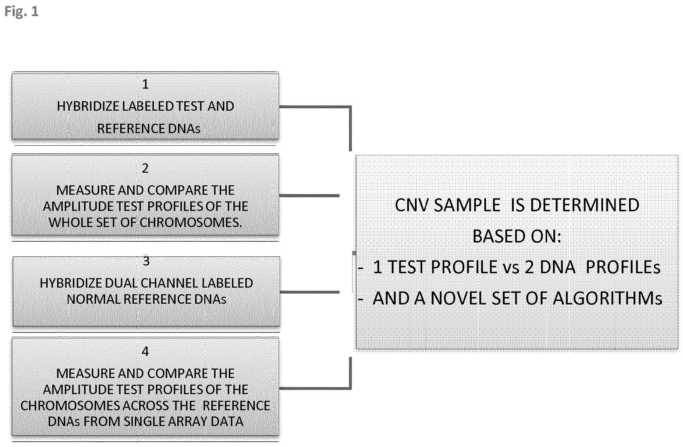

FIG.1 is a flowchart describing a novel CGH microrray method for CNV determination according to the present invention.
FIG.2 is a flowchart describing the method for CNV determination based on the first DNA amplitude profile according to the present invention.
FIG.3 is a flowchart describing the method for CNV determination based on the second DNA amplitude profile according to the present invention.
FIG.4 is a flowchart describing the method for the final CNV determination based on the first and second DNA amplitude profiles according to the present invention.
FIG.5 is a summary describing the method for the CNV determination based on the first and second DNA amplitude profiles using a single area per each test as detailed in the present invention.
FIG.6 is a flowchart describing the method for image quality control.
FIG.7 is a flowchart describing the method for image noise subtraction and application of the algorithms series for first CNV estimation.
FIG.8 is a flowchart describing the method for estimation of the second and final CNV determination.
FIG. 8A is a table comparing the results obtained from a set of CNV analysis, in terms of chromosomes GAIN LOSS, between the test and the male or female reference; the female reference was from both the quality control and the reference measured directly with the microarray. According to the autosomes and gonosomes GAIN and LOSS, the PGS karyotype has been estimated and compared to the Control karyotype. The female references (from REF (a) to (e)) concordance has been compared to the quality control and the respective concordance values has been also reported.
FIG. 8B is the CNV reading of the different normal and non-normal potential genotypes versus a normal male or female reference, referred to the gonosomes.
FIG.9 is a comparison of the Standard deviation of the female reference measured on different microchips. The estimation of the median and the standard deviation of those value is also reported.
FIG.10 is a comparison of the Standard deviation of the tests analysis. The estimation of the median and the standard deviation of those value is also reported.
FIG.11 is the chromosome mapping of the tests versus a control.
FIG.12 is the female reference trend when measuring the tests versus the IBSA quality control.
FIG.13 - is an example of a single area CNV detection according to the invention.

### DETAILED DESCRIPTION OF THE INVENTION

In the present description and claims, unless differently specified, the used terminology shall have the same meaning as usually attributed to them by a person skilled in the sector. Furthermore, throughout this description and claims:
"CGH" means Competitive Genomic Hybridization.

"Test DNA" means the DNA to be analyzed on which, in particular, the number of copy number variations has to be determined.

"Copy number variations" or "CNV" means a form of structural variation, i.e. alterations of the DNA of a genome that results in the cell having an abnormal number of copies of one or more sections of the DNA.

"GAIN/LOSS" accounts for a positive (gain) or negative (loss) values. "Reference DNA" means the DNA to be competitively hybridized with the test DNA on the microarrays used in the present method. "Standard DNA" means the DNA immobilized on the arrays, to which both the test DNA and the reference DNA competitively hybridize.

"Test hybridization array" means the array(s) on which the test DNA sample(s) is(are) hybridized in step c) of the present method. "Reference hybridization array" means the array on which the reference DNAs are hybridized in step h) of the present method. "Arrays" (or "microarrays", names used herein interchangeably), mean a coated surface supporting material (as a glass or plastic slide) onto which numerous molecules or fragments of known sequence usually of single-stranded DNA or protein are attached in a regular pattern for use in biochemical or genetic analysis. In the present method the arrays used have the same structure and composition, irrespective of whether they are meant for hybridizing test DNA or the reference DNAs.

The **test DNA** used in the present method is one derived from any organism, in particular a male or female donor, in which CNV need to be determined. When performing the method of the present invention, the sex of the donor of the test DNA can be known or unknown. The test DNA can carry an increased or decreased number of genomic fragments. The organism from which the test DNA is derived can be e.g. one or more polar body 1 and 2 from oocytes, a blastomers from embryo, spermatozoa, cells derived from one or more of: peripheral blood lymphocytes, fetal blood, chorionic villi, amniotic fluid cells, skin, bone marrow, fetal tissues, lung, liver, solid tumors, or ascites, etc.

The method has been described herein with reference to "a test DNA". However the method object of the invention is not limited to testing single DNAs one by one: it can be performed simultaneously on *n* test DNAs (from the same or different donors) insofar as a suitable CGH support is provided: such a CGH support should include *n* test hybridization arrays on which each of the *n* test DNA is analyzed according to the present method. This embodiment is integral part of the method of the invention. Finally, although suitably devised for testing DNA samples, the present method can be functional for all cases in which dual channel microarray could be applied for.

The **reference DNAs** used in the method can be derived from single donors or, alternatively, from multiple male donors, or multiple female donors; in case of multiple donors, the respective genotypes may be also present at different concentrations. Using a reference DNA with these variations has the advantage of reducing genotype biases. The reference DNA's used in the method can be prepared extemporaneously or be used as commercially available. Accordingly, throughout this application, the term "male reference-DNA" means a reference DNA obtained from one or more male donors; the term "female reference-DNA" means a reference DNA obtained from one or more female donors. The expression "a reference-DNA sexually opposite to" (where "opposite to" is used in opposition to a further reference-DNA) means a reference-DNA obtained from a donor whose sex is opposite to the sex of the donor of the further reference-DNA.

In diagnostic practice, the test DNA to be analyzed is often a single DNA molecule (e.g. one obtained from a single cell). Such a single DNA molecule may be unable to generate sufficiently intense signals for CGH analysis. In these cases, the test DNA molecule can be suitably **amplified** via conventional techniques well-known in the art. Non-limitative examples of amplification techniques are DOP-PCR (diverse random primers strategy), plexysomes technology libraries kit such us Genomeplex (Rubicon Genomics), branched-PCR using Phy polymerase kit (Repli-G, Qiagen), etc. Further examples of amplification techniques are presented in the experimental section of this description. The target degree of amplification should be one capable of providing levels of signal equal or at least similar to those of the reference DNAs.
The need to amplify the reference DNA is less frequent: in fact these are as a rule multi-molecule samples, with a degree of amplification sufficiently high for CGH processing; however the invention does not exclude the possibility of amplifying the reference DNAs, whenever necessitated (always with a view to obtain comparable signals with the test DNA). The amplification of the DNAs (test and/or reference) may be performed in a single step or via separate incremental steps, e.g. two subsequent amplification steps where an appropriate amount of DNA is produced far enough to be labeled through a second step of amplification.

The various steps a) to k) of the present method can be described in detail as follows:

### Step a)

The marker used for marking the test-DNA is typically a fluorescent marker. Non-limitative examples of suitable markers, well-known in the art, are cy3 or cy5 modified dideoxynucleotides (Cy3- cy5-dCTP GE Healthcare or Perkin Helmer). Alternative markers like e.g. colored non-flourescent markers, radiomarkers, etc. are also possible. Labeling can be obtained by known procedure, e.g. Random primed labeling using Klenow fragment of DNA polymerase I of *E. Coli*, or equivalent methods known in the sector.

### Step b)

The reference DNA used in this step can indifferently be either of male or female origin, with no influence on the end-results of the method. However the type of reference DNA used in this step determines the type of reference DNAs used later in steps f) and g). A male reference DNA used in step b) will require using female reference DNAs (thus sexually opposite) in steps f) and g); by converse, a female reference DNA used in step b) will require using male reference DNAs in steps f) and g). The marker used in this step can be chosen among those described in step a), provided that the two markers are different from one another, as required by CGH. Typically, markers with different fluorophores are used, differing in their color/emission wavelength. Preferably the two markers belong to the same family of markers, e.g. "fluorescent", or "radio" in order to enable discrimination of the corresponding labeled DNAs via the same detection instrument. The two markers are suitably used in equal proportions.

The step b) is herein meant broadly, also including the possibility of using a pre-marked commercial reference DNA; in this case the act of physically labeling the reference DNA is replaced by obtaining a pre-labeled marker from a commercial source: this embodiment is meant to be included in step b) of the present method.

### Step c)

This step represents the sole hybridization reaction undergone by the test DNA throughout the entire method: for this reason, the present method is characterized as a "single array" one. At this stage, the labeled DNA's obtained in steps a) and b) (test- and reference) are hybridized competitively on the same test hybridization array. Hybridization is performed by methods known in the art: typically, the labeled DNAs are dissolved into an appropriate hybridization solution; the DNAs are denatured in order to make the single strands available for hybridization with the DNA probes of the array; the resulting solution is then dispensed on the array, thus starting the hybridization reaction. As usual in CGH, the test and reference DNAs compete for hybridization with respect to each of the various DNA clones (BAC / probes) present on the array: any possible test/reference unbalanced hybridization at single BACs signals possible abnormality of the test DNA in its corresponding portion. After suitable incubation time, the array is washed to remove unbound DNA, and dried.

### Step d)

In this step the hybridization levels of the test- and reference DNA are measured by assessing, at each point of the array, the signal intensities of the labeled DNAs bound thereto. A suitable software-assisted reading instrument (e.g. a laser scanner) discriminates and quantifies, for each point of the array, the contribution of the two markers to the signal intensity measured. The resulting signal intensity is recorded and saved in order to be used later in the method.

### Step e)

The CNV in the test DNA are determined by detecting imbalances in hybridization levels in correspondence of specific points of the array; as usual in CGH, imbalances can be detected as loss/gain of emission signals at specific points of the array. In particular, when the log2 ratio between the two signal intensities (test DNA and reference DNA) accounts for a positive (gain) or negative (loss) value, then a CNV can be identified. The calculation of CNV is performed by software processing of the signals, according to techniques well known in the art: these involve e.g. quantification of the signal intensity, data normalization, statistical analysis, bias reduction, calculation of chromosome-related intensities, generation of chromosome-related DNA segments, etc. An example of suitable software for this purpose is the one described in the patent application WO2013/171565 A2. Further details of software and algorithms for this calculation are shown in the experimental section of this description. The chromosome CNV values read in this step is final with respect to non-sexual chromosomes (autosomes), whereas it is provisional as regards sexual chromosomes (gonosomes). This is because when the analyzed test DNA is of unknown sex (i.e. the sex of the donor of said test DNA is unknown), its hybridization with a sole (male or female) reference DNA would not be sufficient to finally discriminate between the CNVs present in normal and eventually abnormal sexual chromosome.

### Step f)

The reference DNA used in this step is sexually opposite to the one used in step b): it will thus be a female/male reference DNA if a male/female reference DNA was used in step b). A first aliquot of this DNA is labeled with a first marker. The marker will preferably be one of the two already used in steps a) and b).

### Step g)

In this step, a second aliquot of the reference DNA used for labeling in step f) is marked with a second marker, different from the first. The marker used here will preferably be the one used in steps a) or b), which has not been chosen for step f).

### Step h)

Hybridization is performed under normal CGH conditions, as described for step c).

### Step i)

The hybridization level of the two reference DNAs on each BACs of the array can be measured by the methods described in step d). The relevant signal intensities are recorded and saved in order to be used subsequently in the method. If these intensities have already been obtained and are available and saved on a suitable data storage means (e.g. from a prior execution of the method, or from a database collection), they can be used directly from this source as a "historical reference DNA hybridization data", avoiding any repeated performance of the sub-sequence of steps f) through i).

### Step i)

Differently from standard CGH, the hybridization in step i) is not followed by determination of CNVs directly on the same hybridization array; this is logical, since in step i) the same reference DNA competes against itself, no significant CNVs should be expected at this stage. The present step j) instead compares the signal intensities obtained in step i) with the signal intensities obtained in step d), and obtains therefrom a second assessment of CNV in the test DNA. The signal intensities to be compared in step j) are those derived from both type of chromosomes, in particular, those deriving from the sexual chromosomes; therefore, any X-gain and Y-loss (or viceversa) present in the signal intensities to be compared will be of particular interest. Step j) compares the hybridization signals of the test DNA with those of both male and female reference DNAs, thus the CNV read at this stage usefully complements the provisional CNV information obtained in step e) via a single-sex reference DNA.

### Step k)

In this software-assisted step, the CNVs obtained in steps e) and j) are combined, to provide a complete and final CNV profile of the whole test DNA subjected to analysis.

As evident from the above, the subsequence of steps a)-e) and the subsequence f)-i) refer to two distinct and independent hybridization assays. The order of performance of these two subsequences as shown above is merely illustrative and not limitative of the present method: the latter can be indifferently performed by inverting their order of, i.e. by performing first steps f)-i), then steps a)-e), and then concluding the method with steps j) and k); this embodiment is meant to be integral part of the method of the present invention.

The method of the invention further includes some preferred embodiments able to further increase the precision of assessment of the CNVs.

According to a first preferred embodiment, the hybridization procedure of the reference DNAs (steps f-g-h-i of the method) is repeated more times using a corresponding number of samples of the same reference DNAs; such repeated hybridizations are performed on microarrays from diverse production batches; then the signal intensities resulting from each hybridization are software-processed in order to eliminate/reduce possible bias and non-specific signals. An exemplified description of such processing is detailed in the experimental section of this description. The so-treated signal intensities are then regarded as "the result of step i)" and processed further according to the method of the invention above described.

According to a second preferred embodiment, the signal intensities obtained from the first embodiment are stored and used as a "Quality Controlled standard" for any further reference DNAs used in the present method. According to this embodiment, the signal intensities originated in step i) from the reference DNA in use are compared with those of the corresponding "Quality Controlled standard", with the purpose of determining/controlling the quality level of reference DNA in use: the more its signal intensities are closer to those of the "Quality Controlled standard", the higher is its quality.

By avoiding duplicated hybridizations of the test DNA with dual-sex reference DNAs, the present method introduces an important simplification in the CNV determination of DNA test samples whose sex is not known beforehand (i.e. when the sex of the donor of said DNA test sample is unknown), such as it the typical case in pre-implantation diagnosis in in-vitro fertilization (IVF) protocols. This simplification translates into a significant cut in the time of analysis (of particular evidence when multiple test DNAs have to be analyzed), as well in reducing the number of CGH arrays required. The determination of CNV allows to choose top oocyte, spermatozoa or the right embryo for the correct chromosome status, etc.

The reduction of arrays underlying the present method, allows to supply new, more compact CHG supports, characterized by comprising, layered upon a suitable support: one or more hybridization arrays for performing said steps a)-e) on one or more test-DNAs, and a single array for performing said step h): the latter could be identified on the support by a suitable printed indication on the support itself, or by placing it in an particular area of the support, making it distinguishable from the area containing the array(s) needed for hybridizing the test DNA(s). These new, more compact supports form as such an additional embodiment of the present invention.

Further object of the disclosure is a kit for performing the method above described, comprising: (a) one or more hybridization arrays for performing said steps a)-e) on one or more test-DNAs; (b) the reference signal intensities of step i) recorded on a suitable data storage support or, in alternative, a reference array for performing said step h) and the required labeled male and female reference DNAs to perform it, optionally associated to a Quality Control of said reference DNAs.

The present disclosure is now further described by reference to the following non-limiting examples.

### EXPERIMENTALS

The following procedure explains the steps to hybridize differentially labeled human probe DNA samples to the human BAC array printed on a coated glass slide. The whole procedure have proven to be reliable in our laboratory and in clinical centers.

Bacterial artificial chromosome (BAC) -arrays, consists of thousands of spots, each of which comprises DNA fragments covering relatively large chromosome regions (about 150-200 kb of size) and is representative of single BAC clone. Each BAC DNA clone is specific to a different chromosomal region and the complete set of clones has been mapped to the respective chromosome locations. Each clone is represented in at least two replica in each array of the microchip slide in order to avoid experimental biases.

For this, BAC-arrays contain a specific human genomic design with a dedicated number of BAC clones, each of which is represented in individual probes and is proven to be good enough for the principal objective of the cell chromosomes screening: the detection of whole chromosomes affected by gains and/or losses.

Chromosomal loss or gain is revealed by the marker adopted by each spot after hybridization of two different DNAs, such as the ratio of fluorescence intensity for the two contrasting marker.

Microarray CGH has been successfully applied for the detection of aneuploidies in single cells after whole genome amplification (WGA) using a single array. For instance, the whole approach have permitted the comprehensive chromosome analysis within the timeframe necessary for oocyte or cleavage stage embryo screening and in addition cell line genetic analysis was successfully achieved.

The microarray has been designed to be used with amplified DNA, wherein the whole genome amplification (WGA) is necessary for samples with poor amounts of genomic DNAs and amplification (WGA) technique is required to obtain enough material for genetic analysis with DNA microarray.

**Study Design** - The object of this study was to verify and to validate the CNV detection method herein described using at least two diverse matrix of euploid or aneuploid samples collected from IVF procedures or cultured cell lines. For the first matrix, a retrospective study was performed collecting a set of samples from the daily routine of clinical IVF centers. The euploid or aneuploidy status of said samples were already predicted by chromosome assessment performed on polar bodies 1 or 2 or blastomers using alternative techniques. For the second, the human lymphoblastoid cell lines carrying one or more known chromosome aberrations was chosen from a public repository for the scope of this study.

**Tests DNAs** were first compared to a male reference DNA in order to estimate a first order of cellular aneuploidy status. For this, a specific set of calling algorithms (algorithms 1 to 6) was applied and the first chromosome CNV was calculated. The spots intensity in the digital file of the DNA-test was stored for the later comparison to the image data of both, the reference female DNA measured in the five microarrays used for this study and the same reference female DNA from Quality Control.

The second measurement of the chromosomes CNV was obtained using the same calling algorithm. Then the two chromosome CNV measurements were compared with a specific algorithm (algorithm 7) so as to calculate and have the overall copy number assessment of the test DNA.

Each of the five reference female DNA (from REF1 to REF5), marked with two contrasting fluorophores, were hybridized on 5 different microarrays. The respective digital file were stored and compared to the stored image of the DNA-tests data set.

The reference female DNA Quality Control is the storage image data obtained from the CGH analysis performed to each microarray batch production and is representative for the highest quality assay of the female or male reference DNA.

For the evaluation of the tests results two parameters were used, the cell ploidy condition and the chromosome concordance. The former define the euploidy or aneuploidy cells status while the latter define the correspondence of the studied chromosomes between the test and the control of known chromosome CNVs.

When using a reference DNA it is assumed that the majority of clone positions is euploid and most of the log2 ratios should vary around zero. DNA, when applied to microarray studies may introduce systematic array-specific bias in the measured log2 ratios, for this normal CNV measurement during array CGH test could led to data misinterpretation.

In order to reduce this phenomenon in this study we provide a "comprehensive normalization" for the correction of the reference DNA array and three data image quality controls steps: quality control parameters of the spots morphology and signal intensity, clones replica Quality Control, clones Quality control.

Briefly, during Quality Control reference DNA is run for each batch array in sexual matched and mismatched conditions and evaluated for specific parameters: % of inclusion clones, experimental standard deviation, signal to noise ratio and CNV capability for the X and Y chromosomes. When a reference DNA is run during a test assay, it is first compared to the corresponding Quality Control reference in order to assess its experimental validity before the following measurements.

In this study each reference DNA is separately measured and compared with the reference Quality Control and experimental standard deviation (SD) is taken into consideration as evaluation parameter.

**Test- and reference DNA Preparation** - For the array, DNA-WGA was obtained with the PicoPlex Single Cell WGA Kit (Rubicon Genomics Inc.) since all the in-house validation studies have been performed using this kit. However for the DNA amplification, any other suitable amplification system can be used because the nature of the amplified product is not so critical as the unamplified product does. Test DNA and reference DNA (male or female) were WGA amplified resulting cut into fragments. The quality of the amplified product was assessed and one out of tenth of the total amplification reaction was labeled and made suitable for CGH array.

**Marking the amplified sample and reference DNAs** - The Random Primed labeling procedure that involves Klenow fragment of DNA polymerase I of E.Coli was used to independently label, with two different markers, both the test and the reference DNA, further resulting cut into fragments. The two said DNAs can be marked for instance using cy3 or cy5 modified dideoxynucleotides and are mixed in equal proportions to perform the CGH test. This allows to acquire, during the chip scanning, two or one composite images respectively related to the DNA sample and control and to measure the signal intensity coming from the competitive hybridization of the two DNA for each spot deposited on the slide.

**Precipitation of marked test- and reference DNAs** - The said marked DNAs are co-precipitated together with a blocking solution in order to avoid any biases coming from the cross hybridization of the highly repetitive sequences which can interfere with the CNV calculation. The marked DNAs are subsequently dried in order to make the marked probes available to the specific hybridization transporting buffer.

**Hybridization of marked test- and reference DNAs** - The dried pellet is dissolved in an appropriate volume of hybridization solution available into the kit. The DNA is denatured in order to make the single strands available to the hybridization process and is dispensed on the microarray allowing the contact with the arrayed probes and the occurrence of the hybridization. The system is a temperature controlled floating hybridization performed in the dedicated cassettes using a water bath. After hybridization, the glass slides are washed with a dedicated stringency in order to remove any unbound marked DNA or any of the unspecific or higher order complexes of nucleic acids that can interfere with the signal amplitude measurements.

**Signal intensity extraction** - After hybridization and washing, the microarray slides can be read and the microarray information are captured and kept as images using a dual laser scanner (Innoscan 710A and following versions, Innopsys). The microarray reading have a dedicated configuration which improves data quality. The laser excites the fluorescent tag and the photomultiplier increases the signal intensity at a dedicated value obtaining a specific ratio value. The intensity of the outcome signal is proportional to the number of target molecules that are bound to each point in the array. The scanner was configured to create two digital image for each channel array, and the information attained from each spot per each channel was stored and further analyzed using software images for the intensity extraction and calculation of each spot.

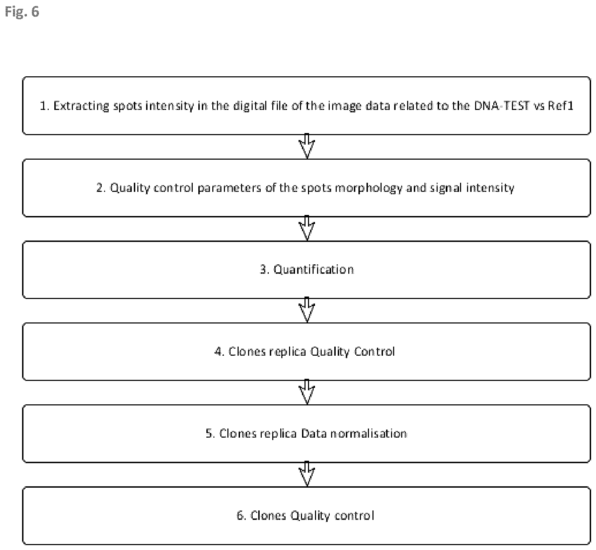

**Data analysis** - The microarray raw data of a set of six test versus male reference DNA hybridizations is obtained and consists of spots listed intensity values per channel and the intensity ratio between channels for each features can be calculated. The intensity ratio of both channels corresponds to the relative abundance of a targeted molecule on each test with respect to a reference DNA. Data interpretation implicates a dedicated statistical methods adapted to microarray analyses and specialized software to visualize these microarray results have been developed. The logical sequence of the main steps is described in (figure 6, 7 and 8).

Details of this procedure are as follows:
The amplitude signals of test and reference hybridizations at each clone position of the microarray are measured, preprocessed and combined as a log2 ratio. This signal is assumed proportional to the log2 ratio of test and reference copy number changes in the corresponding genomic region. If the reference DNAs are chosen euploid, information on copy number changes in the test sample can be gained. The raw data obtained from one CGH-array experiment consists of several thousand clone-specific log2 ratios.

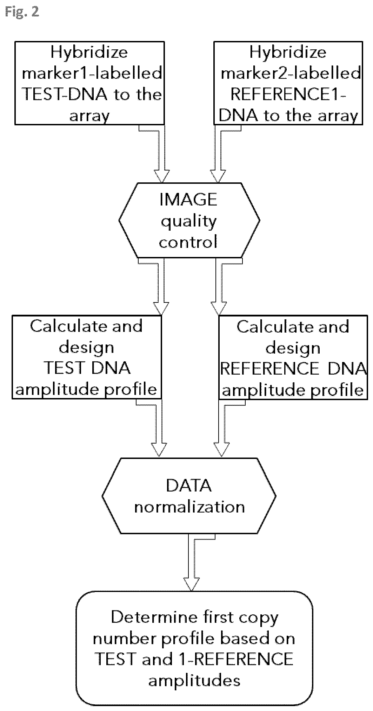

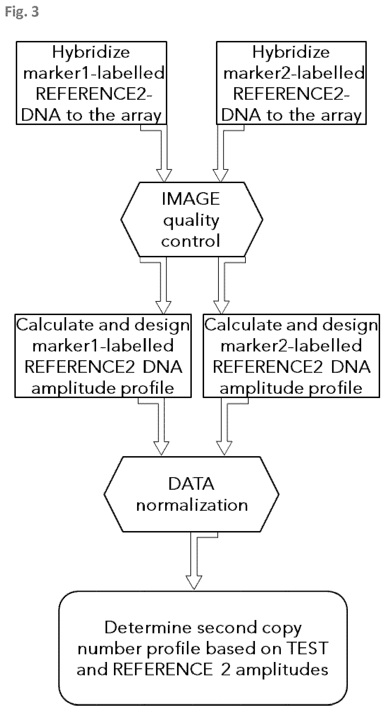

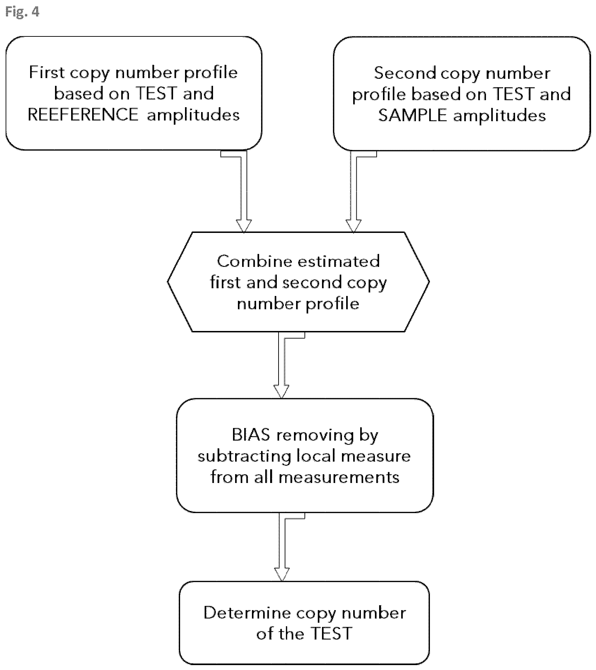

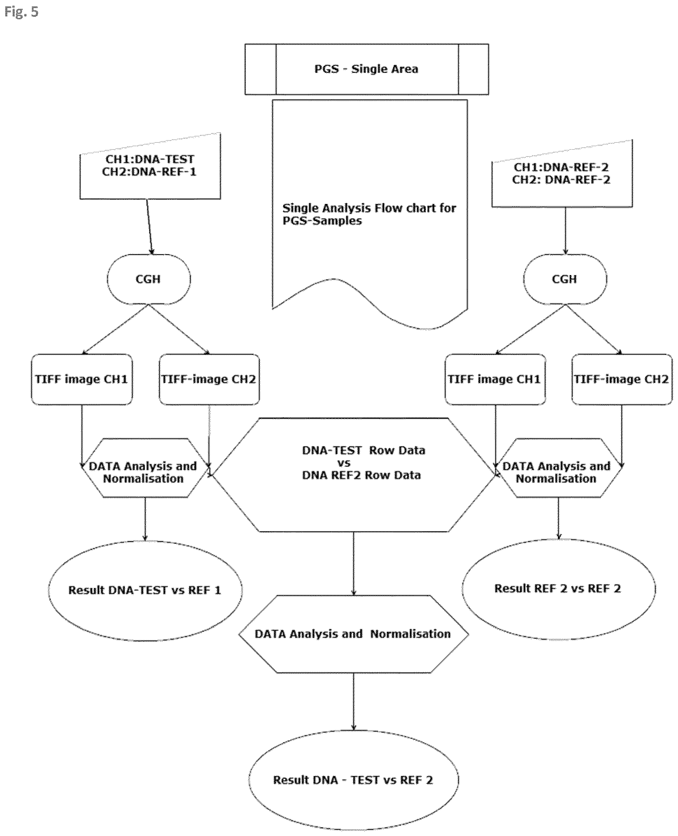

The algorithms implementation of this disclosure can labor single and multi-chip analysis and give support for related statistical investigation. Figure 5 gives a schematic overview over the whole analytical steps and each single step is detailed in figure 2, 3 and 4.

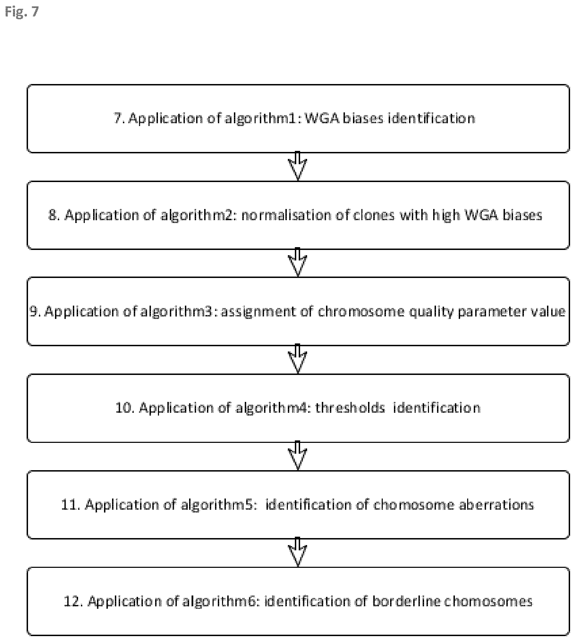

In order to further increase the sensitivity of the method, it is possible to apply image quality control algorithms to calculate and reduce background noise. In this case, following the image intensity extraction, the morphology and spot intensity quality control are performed in three different steps (figure 6) and in figure 7 two diverse algorithms (algorithm 1 and 2) are applied to calculate and reduce the higher noise. We have also implemented a set of specific algorithms aimed at detecting the segments of neighboring clones with the same copy number and for the assignment of a quality parameter value to each chromosome (algorithm 3). The segments obtained are classified into gains, losses and segments with normal copy number. A following algorithm (algorithm4) is applied for the thresholds identification and differentiation within the loss or gain category. Within this technology a fixed threshold is uniformly specified on the log2 ratio scale for all arrays because the application of algorithm 1, 2 and 3 provide a robust noise estimation.

If the segment level is above the threshold is classified as a gain, the segment which is below minus the threshold is counted as a loss and segments at the threshold borderline are classified as warning cases. At this step algorithm 5 and 6 can identify respectively genuine chromosome aberrations and eventually borderline chromosomes (figure 7).

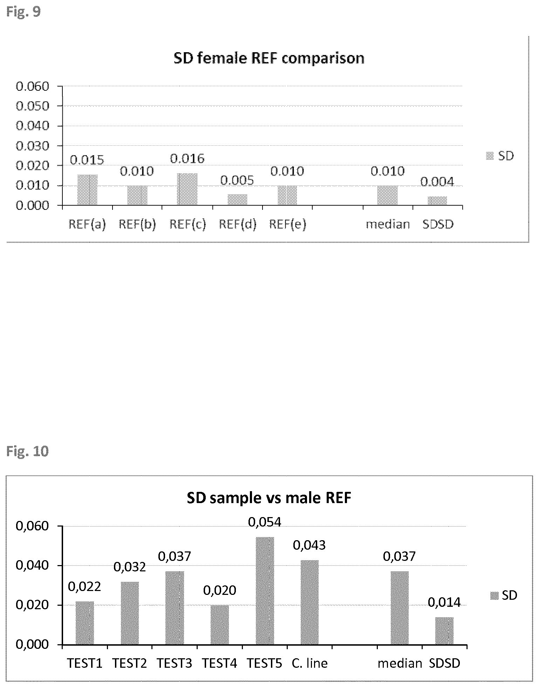

The second reference DNA analysis undergoes to the same image processing: three image quality controls steps followed by the application of the 1 to 6 algorithms. The results of the said quality controls are highlighted in Figure 9 where a comparison between the SD of each female reference experiment with the respective data labels is reported. The overall SD value revealed to be homogeneous and the variation amongst them (SD about 0.004) is very low, this data confirm the accuracy of the reference preparation and makes this procedure robust enough to interpret CGH array of tests with a relevant proportion of aberrant clones.

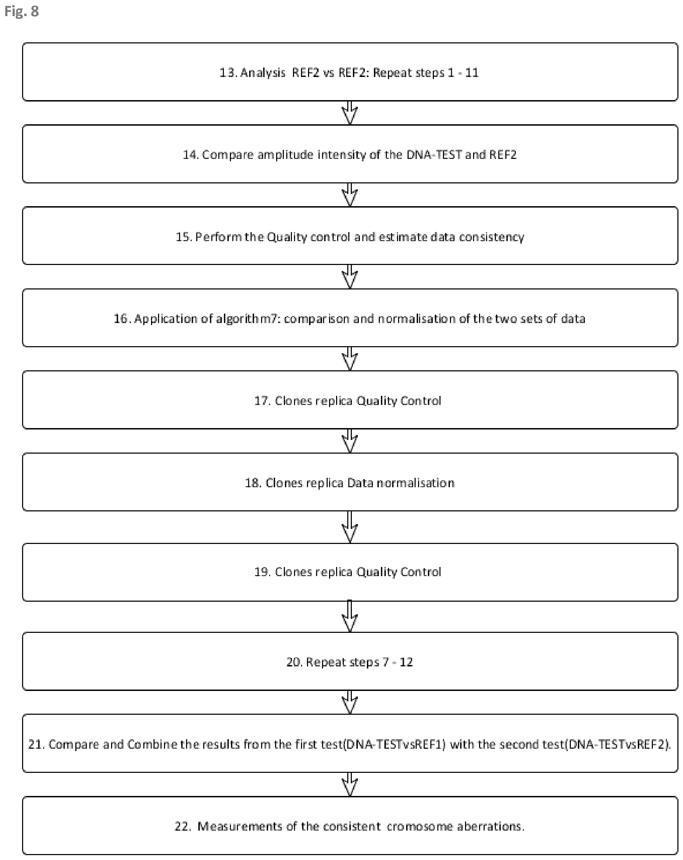

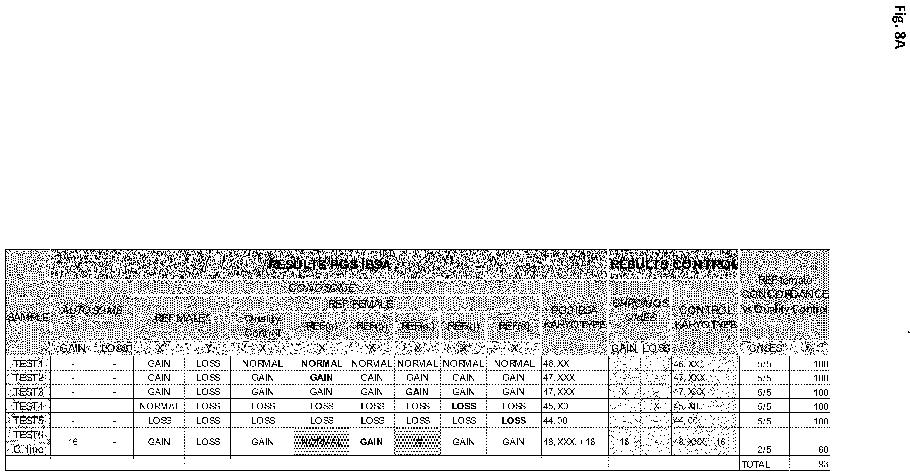

The tests results are shown in the table of Fig. 8A, where the corresponding gain/loss chromosomes analyzed to both male and female reference DNAs are reported. The tests amplitude profile have been compared to the quality control and to five different array hybridizations of the female reference DNA. The respective SD values are described in Figure 10 completed of their median and the overall SD values (0.037 and 0.014 respectively). Although the analysis of the DNA tests has led to divergent values between SDs, the comprehensive normalization of the overall data makes the data reading less error prone and less time-consuming leading to success the overall procedure for CNVs detection. In fact, solely 1 out of 5 cases of the cell line analysis failed the attempt to secure detection despite the other tests.

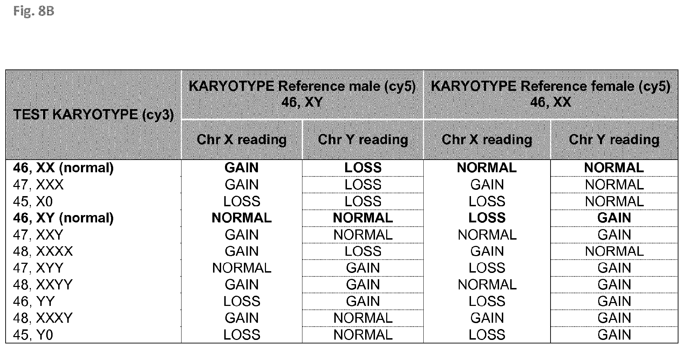

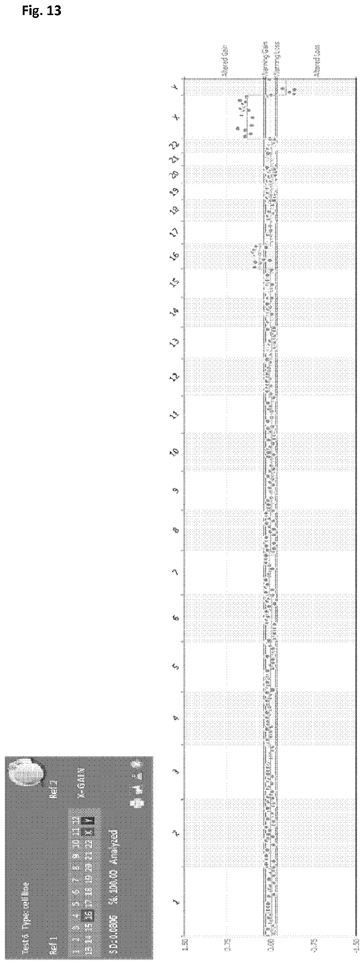

The CNV detection of the DNA test versus the DNA male reference (DNA REF1) (reported as chromosome gain/loss) is then compared with the CNV detection of the DNA test versus the "quality-controlled" DNA female reference or a standard DNA female reference (DNA REF 2), as exemplified in Fig. 8A (in this experiment, for standardization purpose, the hybridization involving the DNA REF 2 was repeated on five separate hybridization arrays, and the corresponding CNV obtained, reported as chromosome gain/loss, are indicated under the headings REF(a)-(e); the determination was performed simultaneously on six test DNAs of different origin, indicated in Fig. 8A as TEST1 to TEST6). This result is achieved through the same image procedure applied for the previous steps in order to obtain two set of data, one related to the DNA test and the second related to the DNA REF2 where a specific algorithm (algorithm 7) compares and normalizes the two sets of data (figure 8).Measurements of the consistent chromosome aberrations is gained through the combination of the provisional results from the first (DNA-test versus DNA reference 1) and both the female quality control and each of the second CGH array (DNA-test versus DNA reference 2). Figure 13 shows a graphical example describing the output of a single area analysis where the test sample is compared to the male reference DNA and in the same windows results of the test sample compared to the female reference DNA is also reported.

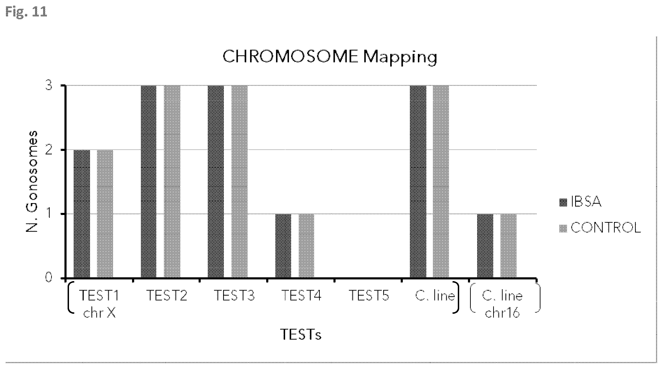

The respective karyotype obtained from the control analysis was compared to the PGS karyotype according to the CNV reading in Fig. 8B, the map chromosomes designed in figure 11 underline the fully correspondence for all the chromosomes studied.

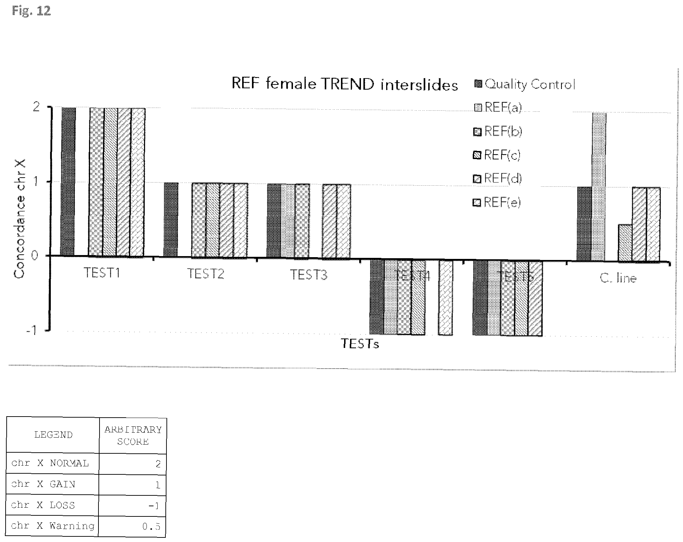

The CNV detection concordances between each set of female reference and quality control was also evaluated and the CNV trend measured in terms of chromosome gain, loss, normal and borderline is shown in figure 12. The results clearly shown 100 % of concordance from test 1 to test 5, while the female REF1 failed to detect the right X chromosome only in one case (cell line) and REF3 showed in the same case the X chromosome detection at the borderline level. Even though this is a phenomenon usually due to the nature of the limphoblastoid cell line the overall concordance was about the 60%.

In conclusion the concordance of the whole experimental set of thirty experiments was 93% and 100% was the concordance referred to the IVF samples.

### REFERENCES

⁽¹⁾ Joo Wook Ahn et al. Array CGH as a first line diagnostic test in place of karyotyping for postnatal referrals - results from four years' clinical application for over 8,700 patients. Molecular Cytogenetics 2013, 6:16
⁽²⁾ Hassold T. et al. The origin of human aneuploidy: where we have been, where we are going Human Mol Genetics. 2007 Aug;16(2):R203-R20
⁽³⁾ Fragouli E. et al. Aneuploidy screening for embryo selection. Semin Reprod Med. 2012 Aug;30(4):289-301
⁽⁴⁾ Yimer, N. and Rosnina. Chromosomal Anomalies and Infertility in Farm Animals: A ReviewY. (2014) Pertanika J. Trop. Agric. Sci. 37 (1): 1-18
⁽⁵⁾ Blazak, W. F., & Eldridge, F.E. (1977). A Robertsonian translocation and its effect upon fertility in Brown Swiss cattle. Journal of Dairy Science, 60(7), 1133-1142.
⁽⁶⁾ S. M. Schmutz et al. Chromosomal aneuploidy associated with spontaneous abortions and neonatal losses in cattle. J Vet Diagn Invest 8:91-95 (1996)
⁽⁷⁾ Popescu, C. P., Bonneau, M., Tixier, M., Bahri, I., & Boscher, J. (1984). Reciprocal translocations in pigs. The Journal of Heredity, 75, 448-452.
⁽⁸⁾ Lear TL, Bailey E. Equine clinical cytogenetics: the past and future. Cytogenet Genome Res. 2008;120(1-2):42-9
⁹ Halnan CR.Equine cytogenetics: role in equine veterinary practice. Equine Vet J. 1985 May;17(3):173-7
⁽¹⁰⁾ Matthew Breen, PhD C.Biol M.I.Biol. Update on Genomics in Veterinary Oncology. Top Companion Anim Med. 2009 August ; 24(3): 113-121. doi:10.1053/j.tcam.2009.03.002.
⁽¹¹⁾ Matthew Breen, PhD C.Biol M.I.Biol, Update on Genomics in Veterinary Oncology. Top Companion Anim Med. 2009 August; 24(3): 113-121.
⁽¹²⁾ Brezina PR. et al. Preimplantation Genetic Screening: A Practical Guide. Clin Med Insights Reprod Health. 2013 Feb 27;7:37-42.
⁽¹³⁾ Robert G. EDWARDS. An astonishing journey into reproductive genetics since the 1950's. Reprod. Nutr. Dev. 45 (2005) 299-306
⁽¹⁴⁾ Norbert Gleicher et al. Preimplantation genetic screening (PGS) still in search of a clinical application: a systematic review. Reproductive Biology and Endocrinology 2014, 12:22
⁽¹⁵⁾ Mastenbroek S, Twisk M, van Echten-Arends J, Sikkema-Raddatz B, Korevaar JC, Verhoeve HR, Vogel NE, Arts EG, de Vries JW, Bossuyt PM, Buys CH, Heineman MJ, Repping S, van der Veen F: In vitro fertilization with preimplantation genetic screening. N Engl J Med 2007, 357:9-17.
⁽¹⁶⁾ Anderson RA, Pickering S: The current status of preimplantation genetic screening: British fertility society policy and practice guidelines. Hum Fertil (Camb) 2008, 11:71-75.
⁽¹⁷⁾ Geraedts J et al. Polar body array CGH for prediction of the status of the corresponding oocyte. Part I: clinical results.
⁽¹⁸⁾ Magli MC. et al. Polar body array CGH for prediction of the status of the corresponding oocyte. Part II:Technical aspects. Hum Reprod. 2011 Aug;16(11):3181-85
⁽¹⁹⁾ Kuliev A, Verlinsky Y: Place of preimplantation genetic diagnosis in genetic practice. Am J Med Genet A 2005, 134A:105-110.
⁽²⁰⁾ Hardarson T, Hanson C, Ludin K, Hillensiö T, Nilsson L, Stevic J, Reismer E, Borg K, Wikland M, Bergh C: Preimplantation genetic screening in women of advanced maternal age causes a decrease in clinical pregnancy rate: a randomized controlled trial. Hum Reprod 2008, 23:2806-2812.
⁽²¹⁾ Mausumi Das, M.D., and Hananel E. G. Holzer, M.D. Recurrent implantation failure: gamete and embryo factors. Fertility and Sterility Vol. 97, No. 5, May 2012.
²² Raziel A, Friedler S, Schachter M, Kasterstein E, Strassburger D, Ron-El R. Increased frequency of female partner chromosomal abnormalities in patients with high-order implantation failure after in vitro fertilization. Fertil Steril 2002;78:515-9.
⁽²³⁾ Ginsburg ES, Baker VL, Racowsky C, Wantman E, Goldfarb J, Stern JE: Use of preimplantation genetic diagnosis and preimplantation genetic screening in the United States: a Society for Assisted Reproductive Technology Writing Group paper. Fertil Steril 2011, 96:865-868.
⁽²⁴⁾ Bassem A. Bejjani and Lisa G. Shaffer. Application of Array-Based Comparative Genomic Hybridization to Clinical Diagnostics. Journal of Molecular Diagnostics, Vol. 8, No. 5, November 2006
⁽²⁵⁾ Stephanie N. Dorman et al. Expanding probe repertoire and improving reproducibility in human genomic hybridization. Nucleic Acids Research, 2013, Vol. 41, No. 7 e81 doi:10.1093/nar/gkt048
⁽²⁶⁾ Pinkel,D., Landegent,J., Collins,C., Fuscoe,J., Segraves,R., Lucas,J. and Gray,J. (1988) Fluorescence in situ hybridization with human chromosome specific libraries: detection of trisomy 21 and translocations of chromosome 4. Proc. Natl Acad. Sci. USA, 85, 9138-9142.
⁽²⁷⁾ Andy W Pang. Towards a comprehensive structural variation map of an individual human genome. Genome Biology 2010, 11:R52
⁽²⁸⁾ Malcolm A. Ferguson-Smith, MBChB, FRCPath, FRS. Cytogenetics and the evolution of medical genetics. Genet Med 2008:10(8):553-559.
⁽²⁹⁾ Conrad,D.F., Pinto,D., Redon,R., Feuk,L., Gokcumen,O., Zhang,Y., Aerts,J., Andrews,T.D., Barnes,C., Campbell,P. et al. (2010) Origins and functional impact of copy number variation in the human genome. Nature, 464, 704-712.
⁽³⁰⁾ Wells D. et al. Use of comprehensive chromosomal screening for embryo assessment: microarrays and CGH. Mol Hum Reprod. 2008 Dec;14(12):703-10
⁽³¹⁾ Vanneste E. et al. New array approaches to explore single cells genomes. Front Genet. 2012 Mar 27;3:44.
⁽³²⁾ Pinkel,D., Segraves,R., Sudar,D., Clark,S., Poole,l., Kowbel,D., Collins,C., Kuo,W.L., Chen,C., Zhai,Y. et al. (1998) High resolution analysis of DNA copy number variation using comparative genomic hybridization to microarrays. Nat. Genet., 20, 207-211.
⁽³³⁾ Gray, J.W. and Pinkel,D. (1992) Molecular cytogenetics in human cancer diagnosis. Cancer, 69, 1536-1542
⁽³⁴⁾ Shinawi,M. and Cheung,S.W. (2008) The array CGH and its clinical applications. Drug Discov. Today, 13, 760-770
⁽³⁵⁾ Vikas Bansal. A statistical method for the detection of variants from next-generation resequencing of DNA pools. Bioinformatics. 2010 Jun 15; 26(12): i318-i324.
⁽³⁶⁾ Janus S Jakobsen. Amplification of pico-scale DNA mediated by bacterial carrier DNA for small-cell-number transcription factor ChIP-seq. BMC Genomics (2015) 16:46.
⁽³⁷⁾ Svetlana A, et al. Microarray-Based Comparative Genomic Hybridization Using Sex-Matched Reference DNA Provides Greater Sensitivity for Detection of Sex Chromosome Imbalances than Array-Comparative Genomic Hybridization with Sex-Mismatched Reference DNA. Volume 11, Issue 3, May 2009, Pages 226-237
⁽³⁸⁾ Tineke E. Buffart, Daniëlle Israeli, Marianne Tijssen, Sjoerd J. Vosse, Alan Mrsic, Gerrit A. Meijer andBauke Ylstra. Across array comparative genomic hybridization: A strategy to reduce reference channel hybridizations. Volume 47, Issue 11, pages 994-1004, November 2008.
⁽³⁹⁾ David Greenstein. Control of oocyte meiotic maturation and fertilization. (December 28, 2005), WormBook, ed. The C. elegans Research Community, WormBook, doi/10.1895/wormbook.1.53.1
⁽⁴⁰⁾ G.L. Harton et al. ESHRE PGD Consortium/Embryology Special Interest Group-best practice guidelines for polar body and embryo biopsy for preimplantation genetic diagnosis/screening (PGD/PGS). Human Reproduction, Vol.0, No.0 pp. 1-6, 2010
⁽⁴¹⁾ Priscila Ramos-Ibeas. An Efficient System to Establish Biopsy-Derived Trophoblastic Cell Lines from Bovine Embryos. BIOLOGY OF REPRODUCTION (2014) 91(1):15, 1-10.
⁽⁴²⁾ Pinto,D., Darvishi,K., Shi,X., Rajan,D., Rigler,D., Fitzgerald,T., Lionel,A.C., Thiruvahindrapuram,B., Macdonald,J.R., Mills,R. et al. (2011) Comprehensive assessment of array-based platforms and calling algorithms for detection of copy number variants. Nat. Biotechnol., 29, 512-520.
⁽⁴³⁾ Dellinger,A.E., Saw,S.-M., Goh,L.K., Seielstad,M., Young,T.L. and Li,Y.-J. (2010) Comparative analyses of seven algorithms for copy number variant identification from single nucleotide polymorphism arrays. Nucleic Acids Res., 38, e105.
⁽⁴⁴⁾ Lai,W.R., Johnson,M.D., Kucherlapati,R. and Park,P.J. (2005) Comparative analysis of algorithms for identifying amplifications and deletions in array CGH data. Bioinformatics, 21, 3763-3770

## Claims

1. A comparative genomic hybridization method to assess the presence and number of copy number variations (CNV) throughout a test DNA, wherein the sex of the donor of said test DNA is unknown, said method comprising the steps of:
a) labeling with a first marker, said test-DNA, thus obtaining a labeled test-DNA;
b') labeling with a second marker, different from the first, a male reference-DNA, thus obtaining a labeled male reference-DNA, or
b") labeling with a second marker, different from the first, a female reference-DNA, thus obtaining a labeled female reference-DNA,
c) hybridizing the DNAs obtained in steps a) and b') or a) and b") to a test hybridization array, thus obtaining a combined pattern of signal intensities from both markers;
d) from the combined pattern of step c), determine the single signal intensities produced by the said first and second marker;
e) perform a first evaluation of CNV in said test-DNA, by comparing against each other the two signal intensities obtained in step d);
f) labeling with said first marker, a reference-DNA sexually opposite to the one used in steps b') or b") respectively, thus obtaining a reference-DNA labeled with said first marker;
g) labeling with said second marker different from the first, said reference-DNA being sexually opposite to the one used in steps b') or b"), thus obtaining a reference-DNA labeled with said second marker;
h) hybridizing both DNAs obtained in steps f) and g) to a reference hybridization array, thus obtaining a combined pattern of signal intensities acquired from both markers;
i) from the combined pattern of step h), determine the single signal intensities produced by the said first and second marker;
j) perform a second evaluation of CNV in said test-DNA, by comparing against each other:
- the signal intensity of said test-DNA labeled with said first marker, as determined in step d),
- with the signal intensity of said reference-DNA labeled with said second marker as determined in step i);
k) perform a software-assisted combination of the CNV values obtained in steps e) and j) to provide a complete and final CNV profile of the whole test DNA subjected to analysis;
wherein, in alternative to performing said steps f) through i), the signal intensities referred in step i), previously obtained by performing said steps f) through i), can be obtained from a data storage support where they were previously recorded, wherein said male reference-DNA has been obtained from one or more male donors, and/or said female reference-DNA has been obtained from one or more female donors
and wherein the method includes only a single step of test DNA hybridization, consisting of step c).

2. The method according to claim 1, wherein said test DNA and/or reference DNA consist of one DNA molecule.

3. The method according to claim 1, wherein said test DNA and/or reference DNA consist of more than one DNA molecules.

4. The method according to claims 1-3 wherein said test-DNA has been obtained from a single or a mixture of cells of the same individual.

5. The method according to claims 1-4, wherein said reference DNAs are a mixture of DNAs with a serial dilution of at least one copy number change in one or more predefined regions of the genome.

6. The method according to claims 1-5, wherein in step j), said signal intensity of said test-DNA labeled with said first marker determined in step d), is one deriving from genotypes showing X-gain and Y-loss.

7. The method according to claims 1-6, wherein in steps e) and j) the evaluation of CNVs is obtained by software-assisted techniques involving: quantification of signal intensity, data normalization, statistical analysis, bias reduction, calculation of chromosome-related signal intensities and generation of chromosome-related DNA segments.

8. The method according to claims 1-7, wherein said reference-DNAs is a mixture of different genotypes at different concentrations.

9. The method according to claims 1-8, wherein, prior to performing said method, said test-DNA has been obtained from a human or animal single cell or biopsy material.

10. The method according to claims 1- 9, being applied to oocytes fertilization and embryo transfer in in-vitro fertilization (IVF) programs.

## Patentansprüche

1. Vergleichendes genomisches Hybridisierungsverfahren zum Bewerten des Vorhandenseins und der Anzahl von Kopienzahlvariationen (CNV) in einer Test-DNA, wobei das Geschlecht des Spenders der Test-DNA unbekannt ist, wobei das Verfahren die Schritte umfasst:
a) Markieren der Test-DNA mit einem ersten Marker, wodurch eine markierte Test-DNA erhalten wird;
b') Markieren einer männlichen Referenz-DNA mit einem zweiten Marker, der sich von dem ersten unterscheidet, wodurch eine markierte männliche Referenz-DNA erhalten wird, oder
b") Markieren einer weiblichen Referenz-DNA mit einem zweiten Marker, der sich von dem ersten unterscheidet, wodurch eine markierte weibliche Referenz-DNA erhalten wird,
c) Hybridisieren der in den Schritten a) und b') oder a) und b") erhaltenen DNAs mit einem Test-Hybridisierungsarray, wodurch ein kombiniertes Muster von Signalintensitäten von beiden Markern erhalten wird;
d) Bestimmen der einzelnen Signalintensitäten, die von dem ersten und zweiten Marker erzeugt wurden aus dem kombinierten Muster aus Schritt c),
e) Durchführen einer ersten Bewertung der CNV in der Test-DNA durch gegeneinander Vergleichen der beiden Signalintensitäten der zwei in Schritt d) erhaltenen Signalintensitäten;
f) Markieren einer Referenz-DNA, die jeweils sexuell entgegengesetzt zu der in Schritt b') oder b" verwendeten ist, mit dem ersten Marker, wodurch eine mit dem ersten Marker markierte Referenz-DNA erhalten wird;
g) Markieren der Referenz-DNA, die jeweils sexuell entgegengesetzt zu der in den Schritten b') oder b" verwendeten ist, mit dem zweiten Marker, der von dem ersten verschieden ist, wodurch eine mit dem zweiten Marker markierte Referenz-DNA erhalten wird;
h) Hybridisieren beider in den Schritten f) und g) erhaltenen DNAs mit einem Referenz-Hybridisierungsarray, wodurch ein kombiniertes Muster von Signalintensitäten erhalten wird, die von beiden Markern stammen;
i) Bestimmen der einzelnen Signalintensitäten aus dem kombinierten Muster aus Schritt h), die von dem ersten und zweiten Marker erzeugt wurden;
j) Durchführen einer zweiten Bewertung der CNV in der Test-DNA durch gegeneinander Vergleichen:
- der Signalintensität der Test-DNA, die mit dem ersten Marker markiert ist, wie in Schritt d) bestimmt,
- mit der Signalintensität der Referenz-DNA, die mit dem zweiten Marker markierten ist, wie in Schritt i) bestimmt;
k) Durchführen einer software-unterstützten Kombination der in den Schritten e) und j) erhaltenen CNV-Werte, um ein vollständiges und endgültiges CNV-Profil der gesamten Test-DNA, die der Analyse unterzogen wurde, zu erhalten;
wobei, alternativ zum Durchführen der Schritte f) bis i), die Signalintensitäten, auf die in Schritt i) Bezug genommen wird, die zuvor durch Durchführen der Schritte f) bis i) erhalten wurden, von einem Datenspeicher erhalten werden können, auf dem sie zuvor aufgezeichnet wurden, wobei die männliche Referenz-DNA von einem oder mehreren männlichen Spendern erhalten wurde, und/oder die weibliche Referenz-DNA von einer oder mehreren weiblichen Spenderinnen erhalten wurde, und wobei das Verfahren nur einen einzigen Schritt der Test-DNA-Hybridisierung umfasst, der aus Schritt c) besteht.

2. Verfahren gemäß Anspruch 1, wobei die Test-DNA und/oder die Referenz-DNA aus einem DNA-Molekül besteht/en.

3. Verfahren gemäß Anspruch 1, wobei die Test-DNA und/oder die Referenz-DNA aus mehr als einem DNA-Molekül besteht/en.

4. Verfahren gemäß den Ansprüchen 1-3, wobei die Test-DNA aus einer einzelnen oder einer Mischung von Zellen desselben Individuums erhalten wurde.

5. Verfahren gemäß den Ansprüchen 1-4, wobei die Referenz-DNAs eine Mischung von DNAs mit einer seriellen Verdünnung von mindestens einer Kopienzahländerung in einer oder mehreren vordefinierten Regionen des Genoms sind.

6. Verfahren gemäß den Ansprüchen 1-5, wobei in Schritt j) die Signalintensität der Test-DNA, die mit dem in Schritt d) bestimmten ersten Marker markiert ist, eine von Genotypen ableitete ist, die X-Gewinn und Y-Verlust zeigen.

7. Verfahren gemäß den Ansprüchen 1-6, wobei in den Schritten e) und j) die Auswertung der CNVs durch software-unterstützte Techniken erhalten wird, die beinhalten:
Quantifizierung der Signalintensität, Datennormalisierung, statistische Analyse, Bias-Reduktion, Berechnung von Chromosomen-bezogenen Signalintensitäten und Erzeugung von Chromosomen-bezogenen DNA-Segmenten.

8. Verfahren gemäß den Ansprüchen 1-7, wobei die Referenz-DNA eine Mischung aus verschiedenen Genotypen in verschiedenen Konzentrationen ist.

9. Verfahren gemäß den Ansprüchen 1-8, wobei die Test-DNA, vor dem Durchführen des Verfahrens, aus einer einzelnen menschlichen oder tierischen Zelle oder aus Biopsiematerial gewonnen wurde.

10. Verfahren gemäß den Ansprüchen 1-9, das bei der Befruchtung von Eizellen und Embryotransfer in In-vitro-Fertilisations-(IVF)-Programmen angewendet wird.

## Revendications

1. Procédé comparatif d'hybridation génomique pour évaluer la présence et le nombre de variations du nombre de copies (CNV) dans un ADN-test, dans lequel le sexe du donneur dudit ADN-test est inconnu, ledit procédé comprenant les étapes consistant à :
a) marquer avec un premier marqueur, ledit ADN-test, obtenant ainsi un ADN test marqué ;
b') marquer avec un second marqueur, différent du premier, un ADN de référence masculin, obtenant ainsi un ADN de référence masculin marqué, ou
b") marquer avec un second marqueur, différent du premier, d'un ADN de référence féminin, obtenant ainsi un ADN de référence féminin marqué,
c) l'hybridation des ADN obtenus dans les étapes a) et b') ou a) et b") à un réseau d'hybridation de test, obtenant ainsi un modèle combiné d'intensités de signal des deux marqueurs ;
d) à partir du motif combiné de l'étape c), déterminer les intensités de signal individuelles produites par lesdits premiers et seconds marqueurs ;
e) effectuer une première évaluation du CNV dans ledit ADN-test, en comparant l'une à l'autre les deux intensités de signal obtenues à l'étape d) ;
f) marquer avec ledit premier marqueur, un ADN de référence sexuellement opposé à celui utilisé dans les étapes b') ou b") respectivement, obtenant ainsi un ADN de référence marqué avec ledit premier marqueur ;
g) le marquage avec ledit second marqueur différent du premier, ledit ADN de référence étant sexuellement opposé à celui utilisé dans les étapes b') ou b"), obtenant ainsi un ADN de référence marqué avec ledit second marqueur ;
h) l'hybrider des deux ADN obtenus dans les étapes f) et g) à un tableau référence d'hybridation, obtenir ainsi un modèle combiné d'intensités de signal acquises à partir des deux marqueurs.
i) à partir du motif combiné de l'étape h), déterminer les intensités de signal uniques produites par lesdits premiers et seconds marqueurs ;
j) effectuer une deuxième évaluation du CNV dans ledit ADN-test, en comparant l'intensité du signal dudit ADN-test marqué avec ledit premier marqueur, tel que déterminé à l'étape d), avec l'intensité du signal dudit ADN de référence marqué avec ledit second marqueur, comme déterminé à l'étape i) ;
k) effectuer une combinaison assistée par logiciel des valeurs de CNV obtenues aux étapes e) et j) pour fournir un profil de CNV complet et final de l'ensemble de l'ADN-test soumis à l'analyse ;
dans lequel, alternativement à la réalisation des étapes f) à i), les intensités de signal mentionnées à l'étape i), précédemment obtenues en réalisant lesdites étapes f) à i), peuvent être obtenues à partir d'un support de stockage de données dans lequel elles ont été précédemment enregistrées,
dans lequel ledit ADN de référence mâle a été obtenu à partir d'un ou plusieurs donneurs mâles, et/ou ledit ADN de référence femelle a été obtenu à partir d'un ou plusieurs donneurs femelles, et
dans lequel la méthode ne comprend qu'une seule étape d'hybridation d'ADN de test, consistant en l'étape c).

2. Méthode selon la revendication 1, dans laquelle ledit ADN-test et/ou ledit ADN de référence consiste en une molécule d'ADN.

3. Méthode selon la revendication 1, dans laquelle ledit ADN d'essai et/ou ADN de référence consiste en plus d'une molécule d'ADN.

4. Méthode selon les revendications 1-3, dans laquelle ledit ADN-test a été obtenu à partir d'une seule cellule ou d'un mélange de cellules du même individu.

5. Méthode selon les revendications 1-4, dans laquelle lesdits ADN de référence sont un mélange d'ADN avec une dilution en série d'au moins un changement de nombre de copies dans une ou plusieurs régions prédéfinies du génome.

6. Méthode selon les revendications 1-5, dans laquelle à l'étape j), ladite intensité de signal dudit ADN-test marqué avec ledit premier marqueur déterminé à l'étape d), est une intensité dérivée des génotypes présentant un gain X et une perte Y.

7. Méthode selon les revendications 1-6, dans lequelle dans les étapes e) et j), l'évaluation des CNV est obtenue par des techniques assistées par logiciel impliquant :1a quantification de l'intensité du signal, la normalisation des données, l'analyse statistique, le biais, la réduction, le calcul des intensités de signal liées aux chromosomes et génération de segments d'ADN liés aux chromosomes.

8. Méthode selon les revendications 1-7, dans laquelle ledit ADN de référence est un mélange de différents génotypes à différentes concentrations.

9. Méthode selon les revendications 1 à 8, dans laquelle, avant de réaliser ladite méthode, ledit ADN-test a été obtenu à partir d'une cellule unique ou d'un matériel de biopsie humain ou animal.

10. Méthode selon les revendications 1 à 9 appliquée à la fécondation d'ovocytes et au transfert d'embryons dans des programmes de fécondation in vitro (FIV).
